Europäisches Patentamt

European Patent Office

Office européen des brevets

(19)

(11) Publication number: **0 012 123**
**B1**

(12) **EUROPEAN PATENT SPECIFICATION**

(45) Date of publication of patent specification: **16.02.83**

(51) Int. Cl.³: **A 61 M 5/14, H 05 B 1/02**

(21) Application number: **79850098.9**

(22) Date of filing: **07.11.79**

(54) **A device for heating a fluid.**

(30) Priority: **22.11.78 SE 7812016**

(43) Date of publication of application:
**11.06.80 Bulletin 80/12**

(45) Publication of the grant of the patent:
**16.02.83 Bulletin 83/7**

(84) Designated Contracting States:
**AT BE CH DE FR GB IT LU NL**

(56) References cited:
**FR - A - 2 330 410**
**FR - A - 2 378 308**
**FR - A - 2 405 610**

(73) Proprietor: **GAMBRO AB**
**Box 10101**
**S-220 10 Lund (SE)**

(72) Inventor: **Dahlberg, Bengt Ake Gustav**
**Masvägen 8c**
**S-222 33 Lund (SE)**
Inventor: **Holmberg, Bengt Magnus**
**Svalvägen 27**
**S-230 50 Bjärred (SE)**
Inventor: **Nilsson, Lennart Olov Erland**
**Furustigen 11**
**S-240 13 Genarp (SE)**

(74) Representative: **Boberg, Nils Gunnar Erik**
**HOLGER CRAFOORD AB Patent Department Box 10101**
**S-220 10 Lund (SE)**

Courier Press, Leamington Spa, England

A device for heating a fluid

Technical Field

This invention relates to a device for heating a fluid flowing in a conduit. Said device comprises electrical heating means, such as electrical heating plates, heating coils or the like, in heat transferring contact with at least part of said conduit, and a temperature transmitter in intimate thermal contact with the conduit at its outlet part and at such a distance from the heating means that the temperature transmitter is essentially uninfluenced by conductive and/or radiant heat emanating therefrom.

The expression "fluid" is intended to cover as well liquids as gases.

Background of the Invention

A device of the above-mentioned kind is described in FR—A—2 330 410.

A serious disadvantage of said known device is that the fluid when flowing in the conduit at small flow rates may find its way past the temperature transmitter without coming in intimate contact therewith at the measuring point. The temperature transmitter will thus sense the surface temperature of the conduit on an improper point rather than in close contact with the flowing fluid and thus indirectly control the current supply to the heating plates on the basis of another temperature than that of the fluid.

An object of the present invention is therefore to provide a device of the above-mentioned kind, which eliminates said serious disadvantage of the known device. More particularly it is an object to provide a device in which the fluid even at small flow rates is caused to flow in intimate contact with the temperature transmitter at the measuring point in such a way that incorrect heat controlling and/or misleading temperature sensing are avoided.

Brief Description of the Invention

The device according to the present invention is characterized by means to adjust the cross-sectional area of the conduit at the position of the temperature transmitter in response to the actual flow rate of the fluid in a manner ensuring that the cross-sectional area of the conduit reduces on reduction of fluid flow thus concentrating the fluid to a flow path in close contact with said temperature transmitter.

Said means may comprise contact means in contact with the conduit, which are provided with one or more grooves, whereby the conduit shapes itself to said grooves when the flow rate is sufficiently low and the fluid flow is associated with said grooves.

Preferably said grooves are provided in the flow direction of the fluid, whereby the pressure drop in the conduit due to bends is avoided. Furthermore, said contact means and/or a member, carrying the temperature transmitter, may be spring-loaded, whereby the flow passage between said components is expandable to let a bigger amount of fluid pass per time unit, when the rate of flow of the fluid increases.

As is apparent from the foregoing, the temperature transmitter is located at the outlet end of the present device at such a distance from said heating plates, heating coils or the like that said temperature transmitter is essentially completely uninfluenced by conductive and/or radiant heat emanating therefrom. For example, said temperature transmitter may be insulated from said heating means through an air pocket · separating the outlet end of the device from the heating zone. Said insulation can also be achieved in other ways, such as through thermal insulation which is adapted to surround the area of temperature measuring and to shield said area from the heating means.

That part of the conduit which is in heat transferring contact with for example the heating plates preferably comprises a thin-walled plastic bag having several flow passages. Said passages are in fluid communication with a common outlet and inlet channel, respectively, to be connected to remaining parts of the conduit. The fluid will thereby flow from said conduit and into the passages of the bag via said common inlet channel and out of said passages and back into the conduit via said common outlet channel. The heating occurs in the passages between the inlet and outlet channel.

For several reasons it is convenient to locate the bag vertically in the present device, the inlet channel being at the bottom and the outlet channel being at the top, such that the fluid during heating will flow in a direction towards the gravitation.

One such reason is that the fluid flow out of the conduit can be immediately interrupted, if the measured temperature is too high. If, on the other hand, the fluid is flowing in the direction of the gravitation, at least the amount of fluid on the level above the outlet channel may flow out of the device due to the gravitation. This is particularly serious if the fluid is constituted by blood or other infusion liquid which is to be inserted into a patient.

According to one preferred embodiment of the invention the bag comprises a part having an extended cross-section. Said part is adapted to be in intimate contact with the temperature transmitter and to constitute the common outlet channel. Said part is thereby sufficiently wide to completely cover the temperature transmitter as well as said contact means.

According to still another preferred embodiment of the invention the present device comprises a second temperature transmitter serving to monitor the temperature of the heated fluid. Said monitoring transmitter is also adapted to abut the bag at the outlet end thereof, but lacks contact means. The requirement of rapidity is not so accentuated as regards the monitoring transmitter in comparison to said temperature transmitter. Yet said monitoring transmitter should guarantee that the current supply to the heating element is interrupted when the temperature of the fluid exceeds a predetermined highermost value.

Said heating elements comprise preferably two heating plates having a thickness of only about 0.5 mm to maintain the rest heat in the plates as low as possible. Said plates are conveniently grounded and fitted with a further protection against overheating. Such a further protection may comprise two bimetal contact breakers which interrupt the current supply to the heating plates, when the temperature thereof is too high. This may occur if the device is connected without bag or fluid. In cases where said fluid is constituted by blood said temperature may for instance be 75°C.

For controlling of the temperature the device may furthermore comprise a computer which is adapted to control the set temperature and the true one and to control on the basis thereof. Furthermore, it is operating with regard to as well the flow as any involved alarming system.

A convenient material of the heating plates is aluminum, while a corresponding convenient material of the bag is polyvinyl chloride (PVC).

For example, the present device comprises a rectangular box with a cap, which are operable and closably provided in relation to each other. In each one of said box and said cap there is provided a current-supplied heating element, which may be insulated from the cover through a polyurethane foam insulation. Said heating elements may be heated by means of self-adhering heating foils which are provided in the form of coils on the back of said plates. The bag is thereby adapted to be located between said two heating elements which completely cover the heating zone of the bag. The outlet channel with the expanded part of the bag, as well as the inlet channel, extend out from the heating zone, whereby the wide part of the outlet channel covers the contact means in the cap. Thereby said part will also cover the member carrying the temperature transmitter, which member is provided in the box in line with the contact means, when the two box halves are closed and locked in this position by means of any suitable locking mechanism.

In placing the bag it is important for the bag to be so positioned that it will not be displaced during the heating. For example notches or the like may be provided in the box as well as in the cap, which notches or the like are adapted to receive and retain corresponding parts of the conduit, which are in fluid communication with the outlet and inlet channel, respectively, of the bag.

A further way to hold the bag comprises threading the bag on a rod or the like in the box or the cap. By designing the bag in such a manner that it can be threaded on the rod from only one direction, for example from below, it is assured that the bag always will be correctly located in the device.

The present invention will be described more in detail in the following with reference to the accompanying drawings showing the most preferred embodiment for heating of an infusate. Yet it should be noted that the invention is not intended to be restricted to only this field of application, but could as well be used for any other fluid, liquids as well as gases, which are to be heated during simultaneous controlling and monitoring of the temperature thereof. Since the present invention primarily is intended for use in heating of infusate, such as blood, it will be described in connection therewith.

Fig. 1 is a side view of the present device showing the box and cap in an open position.

Fig. 2 is a plan view of the device of Fig. 1.

Fig. 3 is a side view of the present device, taken along line III—III in Fig. 2.

Fig. 4 is a section along line IV—IV in Fig. 3, showing in detail the spring-loaded contact means.

Fig. 5 shows a plastic bag according to the present invention, when adapted to be used in the device according to Figs. 1—3.

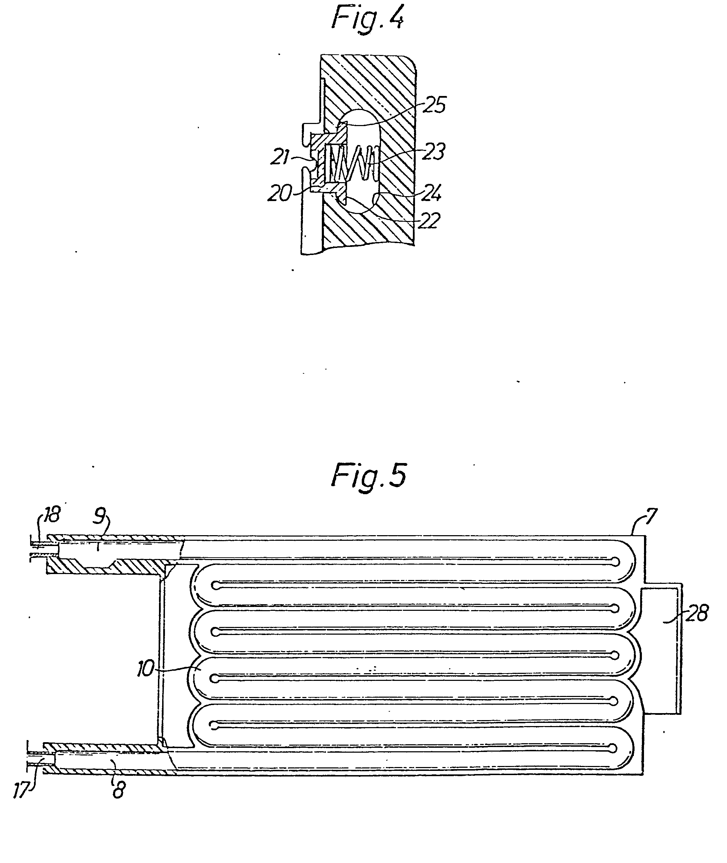

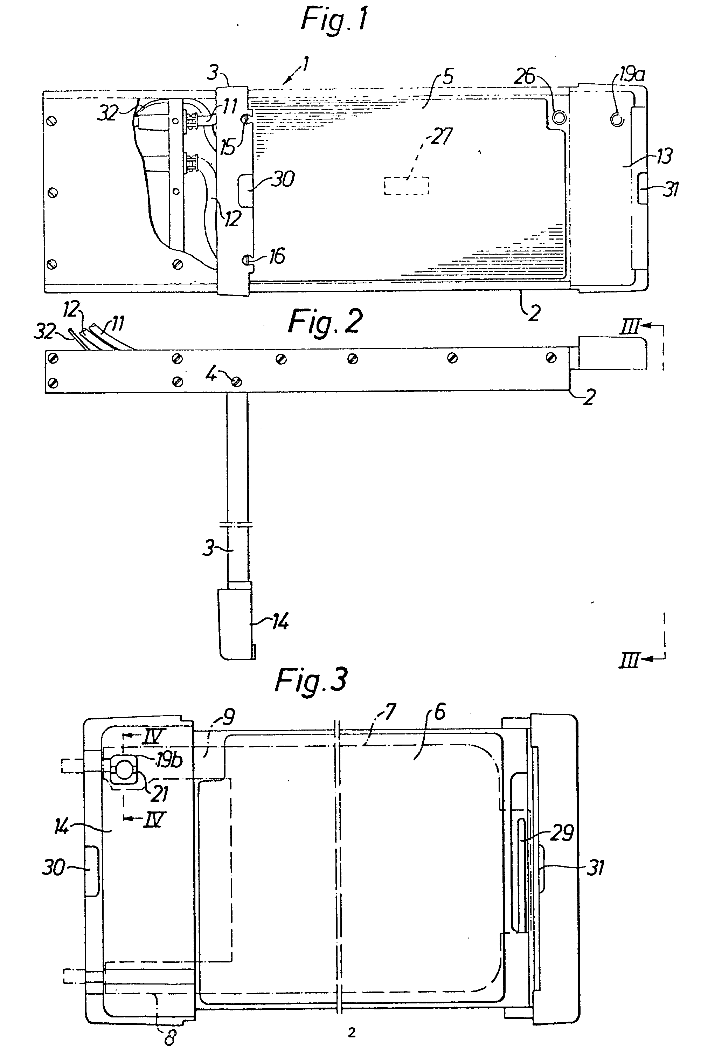

As is shown in Figs. 1 and 2 the present device, generally designated 1, comprises a rectangular box 2 with a rectangular cap 3. The box 2 as well as the cap 3 are made of plastic, wherein said cap is openably and closably mounted at the box, as is shown at 4. The inside of said box is covered with an aluminum plate, while the inside of the cap is correspondingly covered with an aluminum plate 6. Said aluminum plates are thin, about 0.5 mm, and adapted to abut opposing sides of a bag 7 being placed therebetween (Figs. 3 and 5) for heating of an infusate which is caused to flow through passages 8—10 of the bag in a manner to be more closely described in the following. It is to be noted that the bag 7 shown in Fig. 5 differs from the bag schematically shown in Fig. 3. For example, the latter one comprises rounded corners, while the former one has sharp corners. Fundamentally, however, said two bags do not differ from each other. For heating purpose heating foils in the form of coils are attached to the back of each aluminum plate, which foils are supplied with current via lines 11, 12, connected to an outer, not shown, source of current. Said heating foils are thus electrically heated and in turn heat said two aluminum plates 5 and 6. Between the aluminum plates 5 and 6 and the inside of the cap 3 and box 2, respectively, there is provided a thermal insulating material of polyurethane foam.

As is shown in the drawings the box 2 as well as the cap 3 comprise an outlet/inlet part 13, 14 integrally formed with said box and said cap, respectively. Said two parts 13 and 14 lack aluminum plates and form together an air pocket which is shielded from the remaining part of the device, when the box 2 is closed.

Said outlet/inlet part 14 is provided with essentially circular recesses 15 and 16 forming essentially round openings into the inner of the device. Said openings are adapted to receive and by means of snap locking retain corresponding ends 17, 18 of a conduit through which the infusate is flowing.

A temperature transmitter 19a is, as is shown in Fig. 1, provided in the upper right corner of the outlet/inlet part 13, so as to be essentially completely uninfluenced by radiant and/or conductive heat from the two aluminum plates 5 and 6. In a corresponding manner contact means 19b in Fig. 3 are provided in the upper left corner of the outlet/inlet part 14, whereby the bulb of the temperature transmitter 19a will be in an opposing position to the contact means 19b, when the box 2 is closed.

The contact means 19b, shown in detail in Fig. 4, comprise a generally square button 20 having on its top surface a centrally located groove 21 and a peripheral outstanding flange 22 on the bottom surface thereof. The button 20 is thereby spring-loaded (spring 23), which is received in a cavity 24 in the outlet/inlet part 14 and retained against the action of the spring 23 in this cavity through the cooperation between the flange 22 and a corresponding edge 25 of the cavity 24.

A second temperature transmitter 26 (Fig. 1) is similarly provided in the box 2 near the outlet/inlet part 13, but lacks contact means. As is shown in Fig. 1 the temperature transmitters 19a and 26 are in line with each other, whereby the temperature transmitter 26 is located innermost and is carried by the box. Said two temperature transmitters are through lines (line 32) connected to a not shown system which controls the current supply to the aluminum plates 5 and 6 in dependence to measured temperature of the infusate.

As is apparent from the foregoing the temperature transmitter 19a measures the temperature of the infusate which leaves the device, while the temperature transmitter 26 primarily is adapted to interrupt the current supply to the aluminum plates, when the temperature of the infusate exceeds about 42°C. The temperature transmitter 26 serves thereby as an extra safety protection against any over-heating. A further protection against overheating comprises two bimetal breakers (bimetal breaker 27 in Fig. 1), one on the respective backside of the aluminum plates, which are adapted to interrupt the current supply to said plates, when the temperature thereof exceeds 75°C.

The bag 7, as is shown in Fig. 5, comprises an inlet channel 8 and an outlet channel 9 and a flow passage 10 therebetween. The channel 10 extends in a zig-zag path between the channels 8 and 9 and forms together therewith an uninterrupted flow passage for the infusate. The inlet channel 8 as well as the outlet channel 9 form essentially straight extensions of the bag and are adapted to be located completely within said air pocket in the outlet/inlet part, when the box 2 is closed. The flow passage 10 will thereby be completely surrounded by the aluminum plates 5 and 6.

When the bag 7 is placed in the device 1, it is important for the bag to be positioned in the position shown by broken lines in Fig. 3. To this end a rod 29 is provided in the cap 3, which in cooperation with a rod-receiving pocket 28 of the bag permits insertion from only one direction. If the bag is turned up and down, the entrance into said pocket will be turned away from the rod, whereby it is impossible to attach the bag in the device.

The inlet channel 8 is connected to the conduit 17, while the outlet channel 9 is connected to the conduit 18. Also these lines serve to keep the bag in place in the device through snap closing in the openings 15 and 16.

As is schematically shown in Fig. 3 the wider section of the outlet channel 9 will essentially completely cover the top surface of the contact means 19b which by means of the spring 23 and the temperature transmitter 19a thereby will lock the bag when the box is closed. Through this locking infusate which flows with lower flow rates will be concentrated to the groove 21 in close contact with the bulb of the temperature transmitter 19a, whereby an accurate measuring of temperature is assured. At higher rates of flow, on the contrary, the contact means 19b will be pressed against the action of the spring 23 inwardly into the cavity 24, so that a larger amount of infusate per time unit can pass the temperature transmitter 19.

To facilitate the opening and closing, respectively, of the box 2, the cap is provided with a handle in the form of finger grips 30, 31.

Industrial Applicability

The device according to the present invention is adapted for use in controlled heating of a fluid flowing in a conduit. A particular, though not exclusive, field of application involves heating of an infusate, such as blood to be introduced into a patient. Said infusate is thereby caused to flow through flow passages of a bag, which is located in a heating zone of said device, whereby the temperature of the heated infusate is monitored and controlled so as not to exceed 42°. If the temperature for any reason exceeds 42°C, a control system is activated to interrupt the current supply to heating plates of said device. If, on the contrary, said temperature falls essentially below 37°C said control system is similarly activated, whereby the current supply to said heating plates is increased. At local overheating, such as 75°C of the heating plates the current supply is interrupted through bimetal breakers which are adapted to sense the temperature of said heating plates.

Especially the present device is intended for use in heating of infusate from about 15°C to maximum 40°C at a temperature allowance of ±0.5°C at flows of from 15 to 150 ml per minute.

## Claims

1. A device for heating a fluid flowing in a conduit (7, 17, 18), comprising electrical heating means, such as heating plates (5, 6), heating coils or the like, in heat transferring contact with at least part (7) of said conduit, and a temperature transmitter (19a) in intimate thermal contact with the conduit at its outlet part (9) and at such a distance from the heating means (5, 6) that the temperature transmitter (19a) is essentially completely uninfluenced by conductive and/or radiant heat emanating therefrom, characterized by means (19b) to adjust the cross-sectional area of the conduit (7, 17, 18) at the position of the temperature transmitter (19a) in response to the actual flow rate of the fluid in a manner ensuring that the cross-sectional area of the conduit reduces on reduction of fluid flow thus concentrating the fluid to a flow path in close contact with said temperature transmitter (19a).

2. A device in accordance with claim 1, characterized in that said means to adjust the cross-sectional area of the conduit (7, 17, 18) comprises contact means (20) in contact with said conduit, which are provided with one or more grooves (21), whereby the conduit shapes itself to said grooves when the rate is sufficiently low and the fluid flow is associated with said grooves.

3. A device in accordance with claim 2, characterized in that said contact means (20) and/or a member carrying said temperature transmitter are (is) spring-loaded.

4. A device in accordance with claim 1, characterized in that part of said conduit which is in heat transferring contact with said heating plates, heating coils or the like is constituted by a thin, welded plastic bag (7) having one or more flow passages (8—19), wherein the fluid to be heated is caused to flow through said passages.

5. A device in accordance with claim 4, characterized in that it comprises a second temperature transmitter (26) which is adapted to abut the bag and to interrupt current supply to said heating plates, heating coils or the like, when the temperature of the fluid exceeds a certain predetermined value.

6. A device in accordance with claim 1, characterized in that said device comprises a protection (27) against overheating of said heating plates, heating coils or the like, for example in the form of bimetal breakers (27) which are adapted to interrupt the current supply to said means, when the temperature thereof exceeds a critical value.

## Revendications

1. Dispositif pour chauffer un fluide circulant dans un conduit (7, 17, 18), comprenant des moyens de chauffage électriques tels que plaques chauffantes (5, 6), serpentins chauffants ou analogues, en contact d'échange thermique avec une partie au moins (7) dudit conduit, et un capteur de température (19a) en contact thermique intime avec le conduit au voisinage de sa sortie (9) et à une distance des moyens de chauffage (5, 6) telle que le capteur de température (19a) ne soit pratiquement pas influencé par la chaleur par conduction et/ou rayonnement qui en émane, ledit dispositif étant caractérisé en ce qu'il comprend des moyens (19b) pour régler la section transversale du conduit (7, 17, 18) au niveau du capteur de température (19a) en réponse au débit réel du fluide de manière à assurer que la section transversale du conduit diminue avec la réduction du débit, afin de concentrer le fluide en un trajet d'écoulement en contact étroit avec ledit capteur de température (19a).

2. Dispositif suivant la revendication 1, caractérisé en ce que les moyens précités pour régler la section transversale du conduit (7, 17, 18) comprennent des moyens de contact (20) en contact avec ledit conduit, qui comportent une ou plusieurs rainures (21), de façon que le conduit se conforme de lui-même auxdites rainures lorsque le débit est suffisamment bas et que le débit du fluide soit associé auxdites rainures.

3. Dispositif suivant la revendication 2, caractérisé en ce que les moyens de contact précités (20) et/ou un élément supportant le capteur de température soient (est) chargé (s) par un ressort.

4. Dispositif suivant la revendication 1, caractérisé en ce que la partie du conduit qui est en contact d'échange thermique avec les plaques chauffantes précitées, serpentins chauffants ou analogues, est constituée par un mince sac en matière plastique soudée (7) comportant un ou plusieurs passages d'écoulement (8—19), de façon que le fluide à chauffer soit amené à circuler dans lesdits passages.

5. Dispositif suivant la revendication 4, caractérisé en ce qu'il comprend un deuxième capteur de température (26) agencé de manière à porter contre le sac et à interrompre l'alimentation en courant des plaques chauffantes, serpentins chauffants ou analogues, lorsque la température du fluide dépasse une certaine valeur prédéterminée.

6. Dispositif suivant la revendication 1, caractérisé en ce que ledit dispositif comprend une protection (27) contre la surchauffe des plaques chauffantes, serpentins chauffants ou analogues, par exemple sous forme d'interrupteurs bimétalliques (27) qui sont agencés de manière à interrompre l'alimentation desdits moyens lorsque la température de ceux-ci dépasse une valeur critique.

## Patentansprüche

1. Vorrichtung zum Erwärmen eines Fließmittels, welches in einer Leitung (7, 17, 18)
strömt, mit elektrischen Heizeinrichtungen, wie
z.B. Heizplatten (5, 6), Heizspulen oder dergleichen, in Wärmeübertragungsberührung mit
mindestens einem Teil (7) der Leitung und mit
einem Temperaturgeber (19a) in innigem
Wärmekontakt mit der Leitung an seinem
Auslaßteil (9) und in einem solchen Abstand
von der Heizeinrichtung (5, 6), daß der
Temperaturgeber (19a) durch Wärme aufgrund
Leitfähigkeit und/oder Strahlungswärme, die
von dort herströmt, im wesentlichen vollständig unbeeinflußt ist, gekennzeichnet durch
eine Einrichtung (19b) zur Einstellung der Querschnittsfläche der Leitung (7, 17, 18) an der
Stelle des Temperaturgebers (19a) unter An-
sprechen auf die tatsächliche Strömungs-
geschwindigkeit des Fließmittels in solcher
Weise, daß sichergestellt wird, daß die Querschnittsfläche der Leitung sich bei Ver-
ringerung der Fließmittelströmung verkleinert,
wodurch das Fließmittel auf einen Strömungspfad in enger Beruhrung mit dem Temperaturgeber (19a) konzentriert wird.

2. Vorrichtung nach Anspruch 1, dadurch
gekennzeichnet, daß die Einrichtung zur Einstellung der Querschnittsfläche der Leitung (7,
17) 18) Kontakt- bzw. Berührungseinrichtungen (20) in Kontakt mit der Leitung aufweist, die mit einer oder mehreren Nuten (21)
versehen sind, wodurch die Leitung sich selbst
an die Nuten anpaßt, wenn die Geschwindigkeit ausreichend niedrig ist und die Fließmittelströmung den Nuten zugeordnet wird.

3. Vorrichtung nach Anspruch 2, dadurch
gekennzeichnet, daß die Kontakteinrichtungen
(20) und/oder ein den Temperaturgeber tragendes Teil federbelastet sind (ist).

4. Vorrichtung nach Anspruch 1, dadurch
gekennzeichnet, daß der Teil der Leitung, der
sich in Wärmeübertragungskontakt mit den
Heizplatten, Heizspulen oder dergleichen befindet, aus einem dünnen, geschweißten Kunststoffbeutel (7) gebildet ist mit einem oder
mehreren Strömungsdurchgängen (8—19), wobei das zu erwärmende Fließmittel veranlaßt
wird, durch die Durchgänge hindurchzuströmen.

5. Vorrichtung nach Anspruch 4, dadurch
gekennzeichnet, daß sie einen zweiten Temperaturgeber (26) aufweist, der geeignet so ausgestaltet ist, daß er an den Beutel stößt und die
Stromzufuhr zu den Wärmeplatten. Heizspulen
oder dergleichen unterbricht, wenn die Temperatur des Fließmittels einen gewissen vorbestimmten Wert überschreitet.

6. Vorrichtung nach Anspruch 1, dadurch
gekennzeichnet, daß die Vorrichtung einen
Schutz (27) gegen Überhitzen der Wärme-
platten, Wärmespulen oder dergleichen auf-
weist, z.B. in der Form von Bimetallunterbrechern (27), die geeignet ausgestaltet sind,
um die Stromzufuhr zu den Einrichtungen zu
unterbrechen, wenn ihre Temperatur einen kri-
tischen Wert übersteigt.

## Fig.4

25
23
21
24
20
22

## Fig.5

18    9                                                    7

28

10

17    8

**Fig. 1**

**Fig. 2**

**Fig. 3**